(19) Europäisches Patentamt
European Patent Office
Office européen des brevets

(11) Veröffentlichungsnummer: **0 340 408 B1**

(12) **EUROPÄISCHE PATENTSCHRIFT**

(45) Veröffentlichungstag der Patentschrift: **09.12.92**

(51) Int. Cl.5: **C07C 45/64**, C07C 49/84

(21) Anmeldenummer: **89103749.1**

(22) Anmeldetag: **03.03.89**

# (54) Verfahren zur Herstellung von 2-Hydroxy-4-(2'-hydroxyethoxy)-benzophenonen.

(30) Priorität: **30.04.88 DE 3814781**

(43) Veröffentlichungstag der Anmeldung:
**08.11.89 Patentblatt 89/45**

(45) Bekanntmachung des Hinweises auf die Patenterteilung:
**09.12.92 Patentblatt 92/50**

(84) Benannte Vertragsstaaten:
**CH DE FR GB IT LI**

(56) Entgegenhaltungen:
**FR-A- 1 560 184**
**FR-A- 1 592 681**
**FR-M- 1 051**
**US-A- 4 474 951**
**US-A- 4 503 216**

**BULLETIN OF THE CHEMICAL SOCIETY OF JAPAN, Band 46, 1973, Seiten 553-556; TERUO YOSHINO et al.: "Synthetic Studies by the Use of Carbonates. III. The Condensation Reactions of Ethylene Carbonate with a Variety of Phenols Catalyzed by Lithium Hydride or Tetraethylammonium Halides"**

(73) Patentinhaber: **BASF Aktiengesellschaft**
**Carl-Bosch-Strasse 38**
**W-6700 Ludwigshafen(DE)**

(72) Erfinder: **Hahn, Erwin, Dr.**
**Am Buechsenackerhang 31**
**W-6900 Heidelberg(DE)**
Erfinder: **Neumann, Peter, Dr.**
**Poststrasse 28**
**W-6800 Mannheim 31(DE)**

**EP 0 340 408 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von 2-Hydroxy-4-(2'-hydroxyethoxy)-benzophenonen der Formel (I)

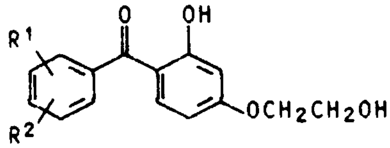

(I),

in der

$R^1$ und/oder $R^2$ für Wasserstoff, Fluor, Chlor, Brom, Cyan, $C_1$- bis $C_8$-Alkyl, $C_4$-$C_7$-Cycloalkyl, durch Fluor, Chlor oder Brom substituiertes $C_1$- bis $C_4$-Alkyl, $C_1$-$C_8$-Alkoxy, Phenoxy, Phenyl, Benzyl, substituiertes Phenoxy, Phenyl oder Benzyl, Hydroxy oder Hydroxyethoxy stehen, durch Umsetzung von 2,4-Dihydroxybenzophenonen der Formel (II)

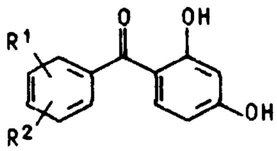

(II),

in der $R^1$ und $R^2$ in obengenannte Bedeutung haben, mit Ethylencarbonat in Gegenwart eines Katalysators bei erhöhten Temperaturen und anschließende Isolierung von (I) aus dem Reaktionsgemisch.

Gemäß der US-PS 3 676 471 kann 2-Hydroxy-4-(2'-hydroxyethoxy)-benzophenon durch Ethoxylierung von 2,4-Dihydroxybenzophenon mit Ethylenoxid oder Ethylencarbonat in Gegenwart von basischen Katalysatoren, vorzugsweise Alkali- oder Erdalkalicarbonaten oder -alkoholaten, hergestellt werden.

Dieses Verfahren weist jedoch eine Reihe von Nachteilen auf. Es erfordert die Verwendung von Lösungsmitteln, insbesondere von Ketonen, wobei wegen des basischen Milieus nur spezielle, weitgehend inerte Ketone eingesetzt werden können wie das teure Diisobutylketon. Ethylencarbonat wird in großem Überschuß eingesetzt und eine relativ hohe Konzentration an Hilfsbase wird verwendet. Nach der beschriebenen Ausführungsform (Beispiel 1) muß das Reaktionsprodukt extrahiert werden, und zwar mit einem ökotoxikologisch nicht unbedenklich Chlorkohlenwasserstoff.

Um Nebenreaktionen, die bei der Verwendung von basischen oder sauren Katalysatoren auftreten, weitgehend zu vermeiden, wurde in der US-PS 4 261 922 und US-PS 4 341 905 vorgeschlagen, die Alkoxylierung von Phenolen mit cyclischen organischen Carbonaten in Gegenwart von Kaliumiodid bzw. generell Alkalimetallhalogenidsalzen als Katalysator unter neutralen Reaktionsbedingungen (pH = 7) vorzunehmen. Gemäß der US-PS 4 310 708 können auch Phosphoniumsalze als Katalysator verwendet werden. Die letztgenannten Verfahren ermöglichen die Umsetzung in Gegenwart oder vorteilhaft Abwesenheit eines inerten organischen Lösungsmittels und weisen gute Selektivitäten auf. Aus ökotoxikologischer Sicht ist jedoch die Verwendung von Phosphoniumsalzen nicht unbedenklich.

Die Verwendung von Tetraethylammoniumhalogeniden als Katalysator zur Alkoxylierung von Phenolen ist aus Bull. Chem. Soc. Jap. 46, 553 (1973) bekannt, allerdings werden nur monofunktionelle Phenole als Ausgangsstoffe eingesetzt.

Der Erfindung lag die Aufgabe zugrunde, ein einfaches, wirtschaftliches und wenig umweltbelastendes Verfahren zur Herstellung der eingangs beschriebenen 2-Hydroxy-4-(2'-hydroxyethoxy)benzophenone (I) durch selektive Ethoxylierung von 2,4-Dihydroxybenzophenonen zu entwickeln.

Demgemäß wurde ein Verfahren zu Herstellung von 2-Hydroxy-4-(2'-hydroxyethoxy)benzophenonen der Formel (I)

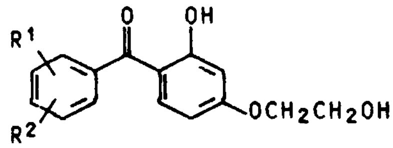

(I),

in der
$R^1$ und/oder $R^2$ für Wasserstoff, Fluor, Chlor, Brom, Cyan, $C_1$- bis $C_8$-Alkyl, $C_4$-$C_7$-Cycloalkyl, durch Fluor, Chlor oder Brom substituiertes $C_1$- bis $C_4$-Alkyl, $C_1$-$C_8$-Alkoxy, Phenoxy, Phenyl, Benzyl, substituiertes Phenoxy, Phenyl oder Benzyl, Hydroxy oder Hydroxyethoxy stehen, durch Umsetzung von 2,4-Dihydroxybenzophenonen der Formel (II)

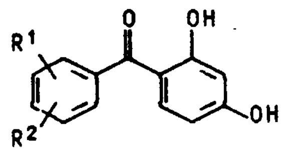

(II),

in der $R^1$ und $R^2$ in obengenannte Bedeutung haben, mit Ethylencarbonat in Gegenwart eines Katalysators bei erhöhten Temperaturen und anschließende Isolierung von (I) aus dem Reaktionsgemisch gefunden, das dadurch gekennzeichnet ist, daß man quartäre Ammoniumsalze als Katalysator verwendet.

Als quartäre Ammoniumsalze eignen sich praktisch alle Verbindungen dieses Typs, die aus Phasentransferreaktionen (s.z.B. E.V. Dehmlow und S.S. Dehmlow, Phase Transfer Catalysis, 2. Aufl. 1983) bekannt sind. Beispielsweise seien Ammoniumverbindungen der Formel (III)

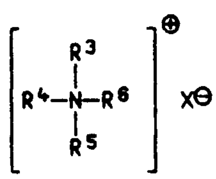

(III)

aufgeführt, in der die Reste $R^3$ bis $R^5$ gleich oder verschieden sind und $C_1$- bis $C_{18}$-Alkylreste bedeuten. Zwei der Reste, z.B. $R^3$ und $R^4$ können darüberhinaus für $C_5$- oder $C_6$-Cycloalkylreste d.h. Cyclopentyl- oder Cyclohexylreste oder für Phenyl stehen. $R^3$, $R^4$ und/oder $R^5$ können auch Benzylreste darstellen. Weiterhin können die Reste $R^3$ und $R^4$ miteinander zu einem 5- oder 6-gliedrigen Ring verknüpft sein und z.B. zusammen mit dem Stickstoff, an den sie gebunden sind, ein Pyrrolidon-, Piperidin-, Pyrol- oder Pyridinsystem bilden.

Als Gegenionen $X^{\ominus}$ in Formel (III) kommen insbesondere die Anionen organischer oder anorganischer Säuren in Betracht. Beispielsweise seien die Anionen $F^{\ominus}$, $Cl^{\ominus}$, $Br^{\ominus}$, $J^{\ominus}$, $CN^{\ominus}$, $SCN^{\ominus}$, $NO_3^{\ominus}$, $OH^{\ominus}$, Acetat, Benzoat oder Sulfonate wie Toluol-4-sulfonat oder Trifluormethansulfonat aufgeführt. Auch weniger nukleophile Anionen wie $HSO_4^{\ominus}$ oder $H_2PO_4^{\ominus}$ kommen prinzipiell in Frage.

Aufgrund der generellen Eignung der Ammoniumsalze richtet sich deren Wahl hauptsächlich nach deren Verfügbarkeit und deren Preis. Praktisch wird man daher vor allem Ammoniumhalogenide, wie -jodide, -bromide oder -chloride verwenden. Wegen ihrer leichten Zugänglichkeit sind Tetraalkylammoniumsalze oder Ammoniumsalze, in denen drei der Reste niedermolekulare Alkylreste und der vierte Benzyl oder $C_6$- bis $C_{18}$-Alkylrest bedeuten, hervorzuheben.

Beispielhaft seien folgende Ammoniumverbindungen genannt:
Benzyldimethyldodecylammoniumbromid, Benzyldimethylhexadecylammoniumchlorid, Benzyldimethyltetradecylammoniumchlorid, Benzyltributylammoniumbromid, Benzyltributylammoniumchlorid, Benzyltriethylammoniumbromid, Benzyltriethylammoniumchlorid, Benzyltriethylammoniumiodid, Benzyltrimethylammoniumbromid, Benzyltrimethylammoniumchlorid, Benzyltrimethylammoniumfluorid, Benzyltrimethylammoniumhydroxid, Benzyltrimethylammoniumethoxid, Dodecylethyldimethylammoniumbromid, Ethylhexadecyldimethylammoniumbromid, Hexadecyltrimethylammoniumbromid, Hexadecyltrimethylammoniumchlorid, Methyltrioctylammoniumbromid, Methyltrioctylammoniumchlorid, Methyltrioctylammoniumiodid, Octadecyltrimethylammoniumbromid, Phenyltrimethylammoniumbromid, Phenyltrimethylammoniumchlorid, Phenyltrimethylammoniumhydroxid, Tetrabutylammoniumbromid, Tetrabutylammoniumchlorid,Tetrabutylammoniumhydroxid, Tetrabutylammoniumiodid, Tetrabutylammoniummethansulfonat, Tetrabutylammoniumrhodanid, Tetrabutylammoniumtoluol-4-sulfonat, Tetrabutylammoniumtrifluoromethansulfonat, Tetradecyltrimethylammoniumbromid, Tetradodecyammoniumbromid, Tetraethylammoniumacetat, Tetraethylammoniumbromid, Tetraethylammoniumchlorid, Tetraethylammoniumcyanid, Tetraethylammoniumfluorid, Tetraethylammoniumhydroxid, Tetraethylammoniumiodid, Tetraethylammoniumrhodanid, Tetraethylammoniumtoluol-4-sulfonat, Tetraethylammoniumtrifluoromethansulfonat, Tetraheptylammoniumbromid, Tetrahexylammoniumbenzoat, Tetrahexylammoniumbromid, Tetrahexylammoniumchlorid, Tetrahexylammoniumiodid, Tetrakis(decyl)ammoniumbromid, Tetramethylammoniumbromid, Tetramethylammoniumchlorid, Tetramethylammoniumhydroxid, Tetramethylammoniumiodid, Tetramethylammoniumtoluol-4-sulfonat, Tetraoctadecylammoniumbromid, Tetraoctylammoniumbromid, Tetrapentylammoniumbromid, Tetrapentylammoniumiodid, Tetrapropylammoniumbromid, Tetrapropylammoniumhydroxid, Tetrapropylammoniumiodid, Tributylheptylammoniumbromid, Tributylmethylammoniumbromid, Tributylmethylammoniumchlorid, Tributylmethylammoniumhydroxid, Tributylmethylammoniumiodid, Trioctylmethylammoniumchlorid, Triethylmethylammoniumbromid.

Die Menge des Katalysators ist nicht besonders kritisch. Im allgemeinen kann man pro Mol Ausgangsstoff (II) 0,1 bis 0,001, insbesondere 0,02 bis 0,05 mol einsetzen. Größere Mengen (z.B. 0,25 mol) sind möglich, aber nicht erforderlich.

Die Reaktionstemperatur kann üblicherweise 100 bis 210, insbesondere 120 bis 200 °C betragen. Zur Erzielung einer befriedigenden Reaktionsgeschwindigkeit sind Temperaturen oberhalb von 140 °C vorteilhaft. Mögliche Nebenreaktionen können unterhalb von 180 °C weitgehend vermieden werden. Als optimaler Temperaturbereich ist daher 140 bis 175 °C anzusehen. In der Regel liegen die Reaktionszeiten dann bei etwa 2 bis 12 Stunden.

Gemäß dem erfindungsgemäßen Verfahren können eine Vielzahl von 2,4-Dihydroxyverbindungen umgesetzt werden. So kann $R^1$ und/oder $R^2$ für Wasserstoff, Fluor, Chlor, Brom, Cyan, $C_1$- bis $C_8$-Alkyl oder Alkoxy wie Methyl, Ethyl, Propyl, Butyl, Isobutyl, tert.-Butyl, Pentyl, Hexyl, Heptyl, Octyl oder die entsprechenden Alkoxyreste stehen. Auch kann $R^1$ und/oder $R^2$ eine durch ein oder mehrere Fluor-, Chlor- oder Bromatome substituierte $C_1$- bis $C_4$-Alkylgruppe, z.B. Trifluormethyl oder 1,2-Dichlorethyl darstellen oder die Bedeutung von Phenoxy, Benzyl und Phenyl haben, wobei die genannten aromatischen Reste inerte Substituenten wie Fluor, Chlor, Brom oder $C_1$- bis $C_4$-Alkyl- oder Alkoxyreste tragen können. Im Hinblick auf die Verwendung der Endprodukte (I) als Lichtschutzmittel besonders bedeutend sind Ausgangsstoffe (II), in denen $R^1$ in ortho-Stellung zur Carboxylgruppe steht und eine Hydroxylgruppe darstellt. Von Bedeutung sind auch Ausgangsstoffe (II), in denen $R^1$ und $R^2$ Wasserstoff oder $R^1$ eine ortho-Hydroxylgruppe und $R^2$ eine para-Hydroxylgruppe darstellen. Bei der erfindungsgemäßen Verwendung des quartären Ammoniumsalzes als Katalysators kann eine Ethoxylierung von ortho-Hydroxylgruppen überraschend weitgehend vermieden werden. Hydroxylgruppen in meta- oder para-Stellung werden dagegen ethoxyliert.

Als Ausgangsstoffe (II) seien beispielsweise aufgeführt:

2,4-Dihydroxybenzophenone, in denen $R^1$ Wasserstoff oder Hydroxy bedeutet und $R^2$ für Wasserstoff, Methyl, Ethyl, Propyl, Isopropyl, Butyl, Methoxy, Ethoxy, Propoxy, O-$C_6H_{13}$, O-$C_8H_{18}$, Phenoxy, Fluor, Chlor, Brom, Cyano, Trifluormethyl oder Benzyl steht, wobei die genannten Substituenten in meta-, ortho- oder para-Stellung zur Carbonylgruppe stehen können. Weitere disubstituierte 2,4-Dihydroxybenzophenone sind z.B. solche, in denen $R^1$, $R^2$ für o-Methyl, p-Phenoxy, m-Methyl, p-Phenoxy, m,p-Dimethyl, m,m-Dimethyl, m,p-Dimethoxy, o,p-Dichlor, m-Chlor, m-Trifluormethyl oder m-Chlor, p-Hydroxy stehen.

Die als Ausgangsstoffe eingesetzten 2,4-Dihydroxyverbindungen (II) sind bekannt oder können auf übliche Weise, beispielsweise durch Friedel-Crafts-Acylierung von Resorcin mit Carbonsäurechloriden der Formel

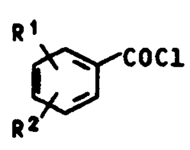 ,

in der $R^1$ und $R^2$ die für Ausgangsstoff (II) genannte Bedeutung haben, hergestellt werden.

Die Umsetzung der 2,4-Dihydroxybenzophenone (II) mit Ethylencarbonat kann in Gegenwart von gegenüber den Reaktanden inerten Lösungsmitteln, beispielsweise Ethern wie Diethylenglykoldimethylether oder Anisol, erfolgen, wird aber bevorzugt lösungsmittelfrei durchgeführt. Ethylencarbonat kann in äquimolaren Mengen zu (II), im Unterschuß oder vorteilhaft in geringem Überschuß eingesetzt werden. Im allgemeinen verwendet man pro Mol Dihydroxybenzophenon (II) 0,95 bis 1,5 mol Ethylencarbonat. Größere Mengen sind möglich, bringen aber keine weiteren Vorteile.

Die Aufarbeitung des rohen Reaktionsgemisches und Isolierung der 2-Hydroxy-4-(2′-hydroxyethoxy)-benzophenone (I) kann in an sich bekannter Weise erfolgen, z.B. durch Zugabe von Wasser oder organischen Lösungsmitteln wie z.B. Alkoholen nach beendeter $CO_2$-Entwicklung und Kristallisation oder Destillation der Endstoffe (I). Auch kann man, falls gewünscht, dem rohen Reaktionsgemisch Aktivkohle oder Bleicherde zusetzen.

Die nach den erfindungsgemäßen Verfahren herstellbaren Produkte (I) eignen sich als Lichtschutzmittel, z.B. zur Lichtstabilisation von organischem Material, speziell von Kunststoffen und Lacken.

Im Hinblick auf ihre Verwendung als Lichtschutzmittel besonders interessante Verbindungen sind:

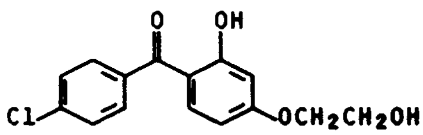

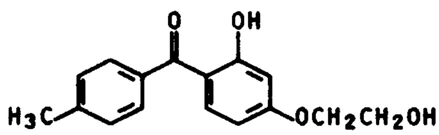

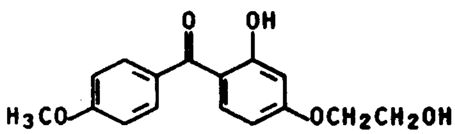

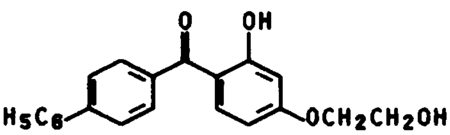

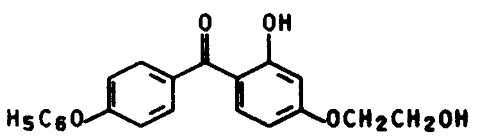

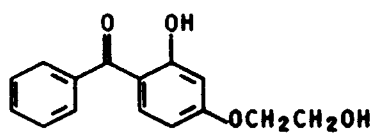

und insbesondere

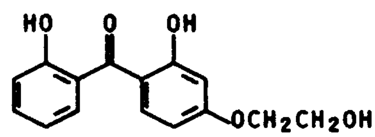

und

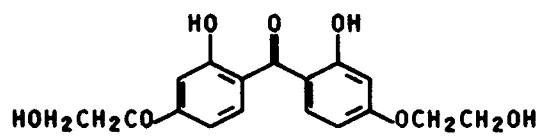

Im allgemeinen fallen die Endstoffe (I) nach dem erfindungsgemäßen Verfahren bereits in ausreichender Reinheit an, es kann jedoch ein weiterer Reinigungsschritt auf einfache Weise mit der Aufarbeitung verbunden werden. 2-Hydroxy-4-(2′-hydroxyethoxy)-benzophenone können z.B. in guter Qualität dadurch erhalten werden, daß man dem Reaktionsgemisch nach beendeter Umsetzung Wasser zusetzt, es mit Alkali z.B. Natronlauge auf einen pH von ca. 10 - 12 einstellt und kurze Zeit (ca. 1 - 2 Stunden) vorteilhaft in der Wärme, z.B. bei Temperaturen von 90 bis 110 °C, nachrührt. Das Reaktionsprodukt (I) wird anschließend durch Ansäuern des Gemisches auf einen pH-Wert von ca. 7 bis 8, z.B. durch Zugabe von Mineralsäure wie verdünnte Salz- oder Schwefelsäure, ausgefällt. Bei Verwendung von Ammoniumiodiden kann es vorteilhaft sein, nach Verdünnen der Reaktion mit Wasser verdünnte Natriumhydrogensulfitlösung zuzusetzen.

Beispiel 1

Ein Gemisch aus 214 g (1 mol) 2,4-Dihydroxybenzophenon, 100 g (1,125 mol) Ethylencarbonat und 5,7 g (0,025 mol) Benzyltriethylammoniumchlorid wurde unter Rühren auf 140 - 150 °C erhitzt, wobei langsam $CO_2$-Entwicklung einsetzte. Nach beendeter $CO_2$-Entwicklung (11 Stunden) kühlte man das Reaktionsgemisch auf ca. 100 °C ab und ließ innerhalb von 5 Minuten 750 ml Wasser unter gutem Rühren einlaufen. Man kühlte unter Rühren auf Raumtemperatur ab, wobei das zunächst noch ölige Reaktionsprodukt kristallisierte. Nach Absaugen des Feststoffes, Waschen mit Wasser und Trocknen erhielt man 2-Hydroxy-4-(2′-hydroxyethoxy)-benzophenon mit einer Reinheit von 97 % (HPLC). Ausbeute: 248 g (≙ 93 % der Theorie).

Beispiel 2

Analog Beispiel 1 erhielt man aus 21,4 g (0,1 mol) 2,4-Dihydroxybenzophenon, 10 g (0,125 mol) Ethylencarbonat und 10 g (0,025 mol ≙ 25 mol%) Tetrabutylammoniumiodid nach einer Umsetzungszeit von 2 Stunden 25,4 g (≙ 98 % d. Theorie) 2-Hydroxy-4-(2′-hydroxyethoxy)-benzophenon.

Beispiel 3

Aus 214 g (1 mol) 2,4-Dihydroxybenzophenon, 100 g (1,125 mol) Ethylencarbonat und 2,28 g (0,01 mol) Benzyltriethylammoniumchlorid erhielt man analog Beispiel 1 nach Verdünnen mit 1000 ml Wasser 232 g (90 % der Theorie) 2-Hydroxy-4-(2′-hydroxyethoxy)-benzophenon.

Beispiel 4

Man verfuhr analog Beispiel 1, arbeitete aber wie folgt auf: Man versetzte das Reaktionsgemisch nach beendeter Reaktion mit 900 ml Methanol und 5 g Aktivkohle, rührte 2 Stunden bei 50 °C, filtrierte und versetzte das Filtrat portionsweise mit 1000 ml Wasser/500 g Eis. Man stellte durch Zugabe von verdünnter Schwefelsäure auf pH 4 bis 5, saugte den Niederschlag ab, wusch ihn mit Wasser und trocknete.
Ausbeute: 200 g (77,5 % der Theorie).

Beispiel 5

Ein Gemisch aus 21,4 g (0,1 mol) 2,4-Dihydroxybenzophenon, 9,7 g (0,11 mol) Ethylencarbonat und 1,9 g (0,005 mol) Hexadecyltrimethylammoniumbromid wurde 11 Stunden auf 150 bis 160 °C erhitzt. Man ließ dann bei ca. 100 °C 75 ml Wasser zulaufen, stellte mit wenig verdünnter Natronlauge auf pH ~ 7,5 - 8 ein, rührte noch 3 Stunden nach und saugte ab. Nach Waschen und Trocknen erhielt man 22 g (85 % der Theorie) 2-Hydroxy-4-(2′-hydroxyethoxy)-benzophenon.

Beispiel 6

214 g (1 mol) 2,4-Dihydroxybenzophenon, 88 g (1 mol) Ethylencarbonat und 9 g (0,025 mol) Tetrabutylammoniumiodid wurden 8 Stunden auf 150 bis 155 °C erhitzt. Die Aufarbeitung erfolgte analog Beispiel 5.
Ausbeute: 237 g (91 % der Theorie).

Beispiel 7

428 g (2 mol) 2,4-Dihydroxybenzophenon, 200 g (2,25 mol) Ethylencarbonat und 22,8 g (0,1 mol) Benzyltriethylammoniumchlorid wurden analog Beispiel 1 umgesetzt. Nach Verdünnen mit 1500 ml Wasser und Abkühlen sowie Rühren stellte man mit verdünnter Natronlauge auf pH ~ 11, rührte 1 Stunde bei 90 bis 100 °C und senkte nach Abkühlen auf Raumtemperatur den pH durch Zugabe von verdünnter Salzsäure auf ca. 7,5 bis 8 ab. Man saugte ab, wusch mit Wasser und trocknete.
Ausbeute: 481 g (93 % der Theorie).

Beispiele 8 bis 11

Entsprechend Beispiel 1 wurden die in folgender Tabelle aufgeführten 2,4-Dihydroxybenzophenone ($R^1$ = H) umgesetzt. Die Produkte sind durch ihre Schmelzpunkte charakterisiert.

Tabelle:

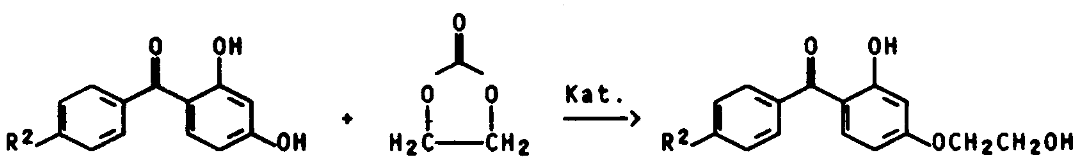


(II) (I)

| Beispiel | $R^2$ | Schmelzpunkt (I) [°C] | Ausbeute[a)] [% der Theorie] |
|---|---|---|---|
| 8 | $CH_3$ | 128 - 130 | 70 (aus Toluol umkristallisiert) |
| 9 | $OCH_3$ | 143 - 146 | 79 (aus Ethanol umkristallisiert) |
| 10 | Cl | 122 - 124 | 91 |
| 11 | $OC_6H_5$ | 96 - 98 | 89 |

a) nicht optimiert

## Patentansprüche

1. Verfahren zur Herstellung von 2-Hydroxy-4-(2'-hydroxyethoxy)benzophenonen der Formel (I)

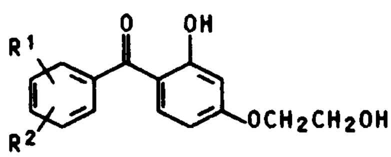

(I),

in der $R^1$ und $R^2$ gleich oder verschieden sind und für Wasserstoff, Fluor, Chlor, Brom, Cyan, $C_1$- bis $C_8$-Alkyl, $C_4$-$C_7$-Cycloalkyl, durch Fluor, Chlor oder Brom substituiertes $C_1$- bis $C_4$-Alkyl, $C_1$-$C_8$-Alkoxy, Phenoxy, Phenyl, Benzyl, substituiertes Phenoxy, Phenyl oder Benzyl, Hydroxy oder Hydroxyethoxy stehen, durch Umsetzung von 2,4-Dihydroxybenzophenonen der Formel (II)

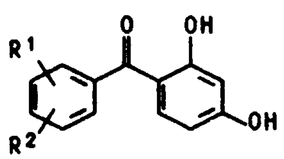

(II),

in der $R^1$ und $R^2$ die obengenannte Bedeutung haben, mit Ethylencarbonat in Gegenwart eines Katalysators bei erhöhten Temperaturen und anschließende Isolierung von (I) aus dem Reaktionsgemisch, dadurch gekennzeichnet, daß man quartäre Ammoniumsalze als Katalysator verwendet.

2. Verfahren nach Anspruch 1, dadurch gekennzeichnet, daß man quartäre Ammoniumsalze der Formel (III)

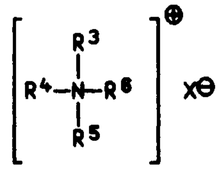

(III),

in der

| | |
|---|---|
| $R^3$, $R^4$ | $C_1$- bis $C_{18}$-Alkyl, $C_5$- oder $C_6$-Cycloalkyl, Phenyl oder Benzyl, |
| $R^5$ | $C_1$- bis $C_{18}$-Alkyl oder Benzyl und |
| $R^6$ | $C_1$- bis $C_{18}$-Alkyl bedeuten oder in der |
| $R^3$ und $R^4$ | miteinander zu einem 5- oder 6-gliedrigen Ring verknüpft sind und |
| $X^\ominus$ | ein anorganisches oder organisches Anionenäquivalent darstellt, |

als Katalysator verwendet.

**3.** Verfahren nach Anspruch 1, dadurch gekennzeichnet, daß man quartäre Ammoniumhalogenide als Katalysator verwendet.

**4.** Verfahren nach Anspruch 1, dadurch gekennzeichnet, daß man Tetraalkylammonium- oder Benzyltrialkylammoniumhalogenide als Katalysator verwendet.

**5.** Verfahren nach Anspruch 1, dadurch gekennzeichnet, daß man 0,1 bis 0,001 mol des quartären Ammoniumsalzes pro Mol Ausgangsstoff II verwendet.

**6.** Verfahren nach Anspruch 1, dadurch gekennzeichnet, daß man die Umsetzung bei Temperaturen von 100 bis 210 °C durchführt.

**7.** Verfahren nach Anspruch 1, dadurch gekennzeichnet, daß man 2,4-Dihydroxybenzophenone (II) umsetzt, deren Reste $R^1$ und $R^2$ Wasserstoff bedeuten.

**8.** Verfahren nach Anspruch 1, dadurch gekennzeichnet, daß man 2,4-Dihydroxybenzophenone (II) umsetzt, in denen sich der Rest $R^1$ in ortho-Stellung zur Carbonylgruppe befindet und eine Hydroxylgruppe darstellt.

**9.** Verfahren nach Anspruch 1, dadurch gekennzeichnet, daß man 2,4-Dihydroxybenzophenone (II) umsetzt, in denen der Rest $R^1$ eine ortho-Hydroxylgruppe und der Rest $R^2$ Wasserstoff oder eine para-Hydroxylgruppe bedeuten.

**10.** Verfahren nach Anspruch 1, dadurch gekennzeichnet, daß man pro Mol 2,4-Dihydroxyverbindung (II) 0,95 bis 1,5 mol Ethylencarbonat verwendet.

## Claims

**1.** A process for preparing 2-hydroxy-4-(2'-hydroxyethoxy)benzophenones of the formula (I)

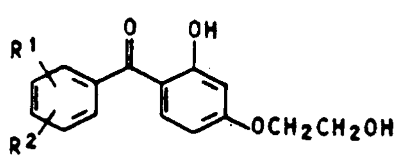

(I),

where $R^1$ and $R^2$ are identical or different and are hydrogen, fluorine, chlorine, bromine, cyano, $C_1$- to $C_8$-alkyl, $C_4$-$C_7$-cycloalkyl, fluorine-, chlorine- or bromine-substituted $C_1$- to $C_4$-alkyl, $C_1$-$C_8$-alkoxy, phenoxy, phenyl, benzyl, substituted phenoxy, phenyl or benzyl, hydroxyl or hydroxyethoxy, by reacting 2,4-dihydroxybenzophenones of the formula (II)

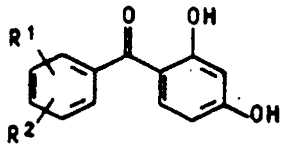

(II),

where $R^1$ and $R^2$ have the abovementioned meaning, with ethylene carbonate in the presence of a catalyst at elevated temperatures and subsequently isolating (I) from the reaction mixture, wherein quaternary ammonium salts are used as catalyst.

**2.** A process as claimed in claim 1, wherein quaternary ammonium salts of the formula (III)

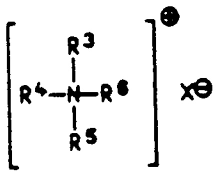

(III),

where

| | |
|---|---|
| $R^3$, $R^4$ | are $C_1$- to $C_{18}$-alkyl, $C_5$- or $C_6$-cycloalkyl, phenyl or benzyl, |
| $R^5$ | is $C_1$- to $C_{18}$-alkyl or benzyl and |
| $R^6$ | is $C_1$- to $C_{18}$-alkyl, or where |
| $R^3$ and $R^4$ | are linked together to give a 5- or 6-membered ring, and |
| $X^{\ominus}$ | is an inorganic or organic anion equivalent, |

are used as catalyst.

**3.** A process as claimed in claim 1, wherein quaternary ammonium halides are used as catalyst.

**4.** A process as claimed in claim 1, wherein tetraalkylammonium halides or benzyltrialkylammonium halides are used as catalyst.

**5.** A process as claimed in claim 1, wherein from 0.1 to 0.001 mol of the quaternary ammonium salt is used per mole of starting material II.

**6.** A process as claimed in claim 1, wherein the reaction is carried out at from 100 to 210 ° C.

**7.** A process as claimed in claim 1, wherein 2,4-dihydroxybenzophenones (II) where $R^1$ and $R^2$ are hydrogen are reacted.

**8.** A process as claimed in claim 1, wherein 2,4-dihydroxybenzophenones (II) where $R^1$ is in the position ortho to the carbonyl group and is hydroxyl are reacted.

**9.** A process as claimed in claim 1, wherein 2,4-dihydroxybenzophenones (II) where $R^1$ is ortho-hydroxy and $R^2$ is hydrogen or para-hydroxy are reacted.

**10.** A process as claimed in claim 1, wherein from 0.95 to 1.5 mol of ethylene carbonate are used per mole of 2,4-dihydroxy compound (II).

**Revendications**

**1.** Procédé de préparation de 2-hydroxy-4-(2'-hydroxyéthoxy)benzophénones de formule (I)

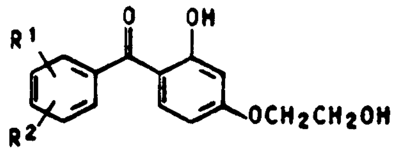

(I),

dans laquelle $R^1$ et $R^2$ sont identiques ou différents et sont mis chacun pour un atome d'hydrogène, de fluor, de chlore ou de brome ou pour un reste cyano, alkyle en $C_1$-$C_8$,cycloalkyle an $C_4$-$C_7$, alkyle en $C_1$-$C_4$ substitué par un atome de fluor, de chlore ou de brome, alcoxy en $C_1$-$C_8$,phénoxy, phényle ou benzyle non substitué, phénoxy, phényle ou benzyle substitué, hydroxy ou hydroxyéthoxy, par réaction de 2,4-dihydroxybenzophénones de formule (II)

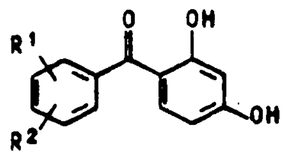

(II),

dans laquelle $R^1$ et $R^2$ ont les significations sus-indiquées, avec du carbonate d'éthylène en présence d'un catalyseur à température élevée, suivie d'isolement de (I) d'avec le mélange réactionnel, caractérisé en ce qu'on utilise, comme catalyseur, des sels d'ammonium quaternaire.

2. Procédé selon la revendication 1, caractérisé en ce qu'on utilise, comme catalyseur, des sels d'ammonium quaternaire de formule (III)

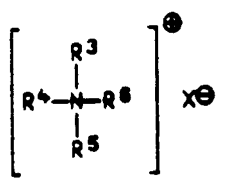

(III),

dans laquelle

| | |
|---|---|
| $R^3$, $R^4$ | représentent des restes alkyle en $C_1$-$C_{18}$, cycloalkyle en $C_5$ ou $C_6$, phényle ou benzyle, |
| $R^5$ | représente un reste alkyle en $C_1$-$C_{18}$ ou benzyle et |
| $R^6$ | représente un reste alkyle en $C_1$-$C_{18}$ |
| | ou dans laquelle |
| $R^3$ et $R^4$ | sont reliés entre eux en un noyau penta- ou hexagonal, et |
| $X^{\ominus}$ | représente un équivalent d'anion inorganique ou organique. |

3. Procédé selon la revendication 1, caractérisé en ce qu'on utilise, comme catalyseur, des halogénures d'ammonium quaternaire.

4. Procédé selon la revendication 1, caractérisé en ce qu'on utilise, comme catayseur, des halogénures de tétraalkylammonium ou de benzyltrialkylammonium.

5. Procédé selon la revendication 1, caractérisé en ce qu'on utilise de 0,1 à 0,001 mole du sel d'ammonium quaternaire par mole de substance de départ II.

6. Procédé selon la revendication 1, caractérisé en ce qu'on conduit la réaction à des températures de 100 à 210 °C.

7. Procédé selon la revendication 1, caractérisé en ce qu'on met en réaction des 2,4-dihydroxybenzophénones (II) dont les restes $R^1$ et $R^2$ représentent des atomes d'hydrogène.

8. Procédé selon la revendication 1, caractérisé en ce qu'on met en réaction des 2,4-dihydroxybenzophénones (II) dans lesquelles le reste $R^1$ se trouve en position ortho par rapport au groupement carbonyle et représente un groupement hydroxyle.

9. Procédé selon la revendication 1, caractérisé en ce qu'on met en réaction des 2,4-dihydroxybenzophénones (II) dans lesquelles le reste $R^1$ est un groupement hydroxyle en position ortho et le reste $R^2$ est un atome d'hydrogène ou un groupement hydroxyle en position para.

10. Procédé selon la revendication 1, caractérisé en ce qu'on utilise, par mole de composé 2,4-dihydroxylé (II), de 0,95 à 1,5 mole de carbonate d'éthylène.